# EUROPÄISCHE PATENTSCHRIFT

(11) **EP 0 908 158 B1**
(45) Veröffentlichungstag und Bekanntmachung des Hinweises auf die Patenterteilung: **29.11.2006**
(21) Anmeldenummer: 98116719.0
(22) Anmeldetag: 03.09.1998
(51) Int. Cl.: A61F 2/36, A61B 17/16

(54) **Satz aus Prothesen unterschiedlicher Grösse**
Set of prostheses of different sizes
Jeu de prothèses de dimensions différentes

(30) Priorität: 16.09.1997 DE 19740755
(43) Veröffentlichungstag der Anmeldung: 14.04.1999
(73) Patentinhaber: UNIOR BIONIC d.o.o., 3214 Zrece (SI)
(72) Erfinder: Copf, Franz, 70178 Stuttgart (DE)
(74) Vertreter: Ostertag, Reinhard

(56) Entgegenhaltungen:
- EP-A- 0 634 145
- EP-A- 0 672 396
- EP-A- 0 733 343
- FR-A- 2 629 707
- FR-A- 2 641 462
- FR-A- 2 662 932
- FR-A- 2 728 160
- US-A- 4 938 771
- US-A- 5 089 004

## Beschreibung

Die Erfindung betrifft einen Satz aus Prothesen unterschiedlicher Größe gemäß dem Oberbegriff des Anspruches 1.

Von Prothesenherstellern werden Sätze von Prothesen unterschiedlicher Größe bereitgehalten, um der unterschiedlichen Größe von Knochen, die bei unterschiedlichen Patienten angetroffen werden, Rechnung zu tragen. Der Operateur wählt jeweils aus dem ihm zur Verfügung gestellten Satz die im Einzelfall passendste Prothese aus.

Auf dem Markt gibt es ferner zwei grundsätzlich unterschiedliche Klassen von Prothesen, nämlich sogenannte Schaftprothesen, die unter Verwendung eines Zementes mit der Wand einer im Knochen erzeugten Kavität verbunden werden, und sogenannte zementfreie Prothesen, bei welchen der in das Knochenende eingeführte Verankerungsabschnitt Hohlräume oder Öffnungen aufweist, in welche Spongiosa im Heilungsprozeß einwachsen kann. Welchen dieser beiden Grundtypen der Operateur im Einzelfalle verwendet, muß vor der Operation festgelegt werden.

Für manche Operationen ist es wünschenswert, wenn man noch im Verlaufe der Operation je nach den angefundenen Verhältnissen die Entscheidung zwischen zementierter Prothese und zementfrei implantierter Prothese treffen könnte.

Die FR-A-2 662 932 offenbart einen Satz von zwei Prothesen gemäß dem Oberbegriff des Anspruchs 1, von denen die eine zementfrei, die andere unter Verwendung von Zement implantiert wird. Die zementrierte Prothese hat einen langen, auf der Außenfläche im wesentlichen glatten Schaft. Die zementfrei zu implantierende Prothese hat die gleiche Geometrie, wobei jedoch auf der Oberfläche noch Rauhigkeiten vorgesehen sind, die Verankerungsmöglichkeiten für gegen die Prothese wachsende Spongiosa bilden.

In der FR-A- 2 728 160 ist ebenfalls ein Paar von Prothesen offenbart, von denen die eine zementfrei, die andere unter Verwendung von Zement implantiert wird. Auch hier haben beide Prothesen im wesentlichen dieselbe Außenkontur, wobei jedoch die glatte Oberfläche der mit Zement zu implantierenden Prothese bei ihren Hauptflächen gegenüber den Hauptflächen der zementfrei zu implantierenden Prothese nach innen versetzt ist. Die zementfrei zu implantierende Prothese trägt auf ihrer Außenseite Rippen, welche sich in das Knochengewebe beim Implantieren hineinschneiden. Durch die Beabstandung der Hauptflächen der zementierten Prothese wird erreicht, daß dann, wenn man die Ausnehmung im Semur unter Verwendung einer einzigen Standard-Raspel herstellt, zwischen den Hauptflächen der zementierten Prothese und der mit der Raspel erzeugten Knochenausnehmung ein Zwischenraum verbleibt, der mit einer Zementschicht gefüllt wird.

Die FR-A-2 629 707 offenbart einen Satz von Prothesen, welche sich in ihrer Geometrie und ihren Abmessungen unterscheiden. Zu diesem Satz von Prothesen gehören Prothesen mit glattem Schaft, wie sie für das Implantieren unter Verwendung von Zement verwendet werden.

Die EP-A-0 672 396 offenbart eine zementierte Prothese, bei welcher ein oberer proximaler Abschnitt gegenüber einem unteren distalen Abschnitt verkippt ist.

Die FR-A-2 641 452 offenbart eine Prothese, die mit oder ohne Zement implantierbar ist, wobei die zementfrei zu implantierbare Prothese in ihrem oberen Abschnitt mit einer Hydroxylapatitschicht versehen wird, ansonsten der zementierten Prothese entspricht.

Die US-A-4 938 771 offenbart eine Prothese, welche in ihrem oberen Abschnitt als Fachwerk ausgeführt ist. Auf diese Weise soll erreicht werden, daß der Prothesenschaft ein ähnliches elastisches Verhalten aufweist wie der natürliche Knochen.

In der EP-A-0 634 145, der EP-A-0 733 343 und der US-A-5 089 004 sind Formraspeln beschrieben, die dazu dienen, im Femur eine Ausnehmung zu erzeugen, in welche eine Femur-Prothese einsetzbar ist.

Durch die vorliegende Erfindung soll ebenfalls ein Satz von Prothesen angegeben werden, mit dem wahlweise ein ein zementfreies Implantieren und ein Implantieren unter Verwendung von Zement möglich ist.

Zur Lösung dieser Aufgabe wird durch die Erfindung ein Satz aus Prothesen unterschiedlicher Größe vorgeschlagen, der die im Anspruch 1 angegebenen Merkmale aufweist.

Erfindungsgemäß steht dem Operateur somit für jede Standardgröße ein Paar alternativ verwendbarer Prothesen zur Verfügung, von denen die eine dem zementierten Typ, die andere dem zementfreien Typ entspricht. Diese beiden Prothesen haben gleiche Grundgeometrie, wobei aber die lichte Außenkontur des Verankerungsabschnittes der zementfreien Prothese über der lichten Außenkontur der zementierten Prothese liegt; der Abstand zwischen diesen beiden Konturen entspricht in der Praxis etwa der beim Einzementieren der zementierten Prothese gewünschten Dicke der Zementschicht zwischen Innenfläche der Knochenkavität und Außenfläche des Prothesenschaftes.

Für beide Prothesen sind somit die gleiche Resektion der Corticalis des mit der Prothese zu verbindenden Endabschnittes des Knochens vorzunehmen, und auch das Herstellen der Kavität erfolgt im wesentlichen (abgesehen von einer etwaigen feinen Nachbearbeitung) gleich.

In der Regel wird für eine Erstversorgung eines Knochens mit einer Prothese die zementfreie Prothese zu bevorzugen sein, da sie nach dem Einheilen die natürliche Struktur eines gesunden Knochens am nächsten abbildet. Hat ein Operateuer nun die entsprechende Präparation des Knochenendes vorgenommen, so kann er dann, wenn er hierbei feststellt, daß für die gerade durchgeführte Operation die zementfreie Prothese doch weniger gut geeignet ist als die zementierte Prothese, problemlos anstelle der an sich vorgesehenen zementfreien Prothese eine zementierte Prothese einsetzen.

Vorteilhafte Weiterbildung der Erfindung sind in Unteransprüchen angegeben.

Die im Anspruch 2 angegebenen Abstände zwischen den lichten Außenkonturen der zementfreien Prothese und der zementierten Prothese ergeben besonders vorteilhafte Einbaubedingungen und besonders vorteilhafte Dicken von Zementschichten.

Die Weiterbildungen der Erfindung gemäß dem Ansprüchen 3 bis 6 haben Geometrien der Verankerungsabschnitte zum Gegenstand, welche sich besonders gut der Corticalis eines Oberschenkelknochens derart anpaßt, daß zwischen der lichten Außenkontur des Verankerungsabschnittes der Prothese und der Innenfläche der durch die Corticalis begrenzten Kavität ein im wesentlichen über die Kavität hinweg konstanter Abstand besteht.

Verwendet man zur Endbearbeitung der Innenfläche der den Verankerungsabschnitt der einzugliedernden Prothese aufnehmenden Kavität ein Werkzeug, wie es im Anspruch 7 angegeben ist, so ist einerseits gewährleistet, daß bei Verwendung einer zementfreien Prothese eine optimale Dicke der zum Einzementieren verwendeten Zementschicht erhalten wird, andererseits bei Eingliederung einer zementfreien Prothese diese in sattem Reibschluß in der Kavität sitzt.

Die vorgenannte Vorteile werden in der Praxis bevorzugt mit Verhältnissen zwischen den lichten Außenkonturen der beiden Prothese und des Werkzeuges erhalten, wie sie im Anspruch 8 angegeben sind.

Die Ansprüche 9 und 10 geben Werkzeuge an, die eine konturgenaue Endbearbeitung der Kavität gewährleisten, andererseits sehr einfach herstellbar sind.

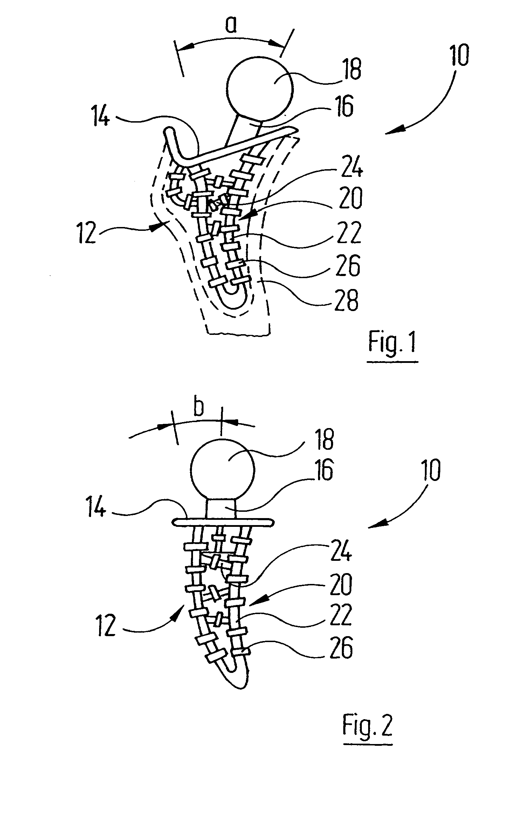

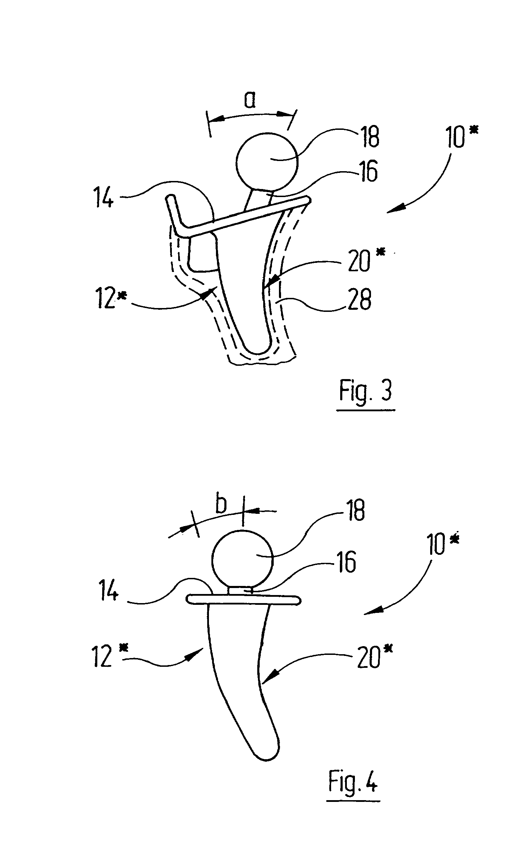

Nachstehend wird die Erfindung anhand von Ausführungsbeispielen unter Bezugnahme auf die Zeichnung näher erläutert , wobei nur die Figuren 1 und 3 die erfindungsgemäßen Merkmale der in den Trochander major ragenden Abschnitt zeigen. Es zeigen:
- Figur 1:: eine seitliche Ansicht einer zementfrei zu implantierenden Hüftgelenksprothese gesehen in Richtung von ventral nach dorsal;
- Figur 2:: eine seitliche Ansicht der Prothese nach Figur 1 in Richtung von lateral nach medial (in Figur 1 von rechts nach links);
- Figur 3 und 4:: Ansichten die denjenigen nach den Figuren 1 und 2 entsprechen, wobei jedoch eine einzuzementierende Prothese wiedergegeben ist;
- Figuren 5: und 6: den Figuren 1 und 2 bzw. 3 und 4 entsprechende Ansichten, in welche die lichten Außenkonturen der zementfrei zu implantierenden Prothese und der einzuzementierenden Prothese übereinander gelegt sind und ferner eine dazwischen liegende lichte Außenkontur eines Werkzeuges gezeigt ist, welches zur Endbearbeitung der die Prothese aufnehmenden Knochenkavität dient;
- Figur 6:: eine Aufsicht auf einen Teil der Oberfläche eines Endbearbeitungswerkzeuges vom Raspeltyp; und
- Figur 7:: eine ähnliche Ansicht wie Figur 6, in welcher jedoch ein Endbearbeitungswerkzeug vom Hobeltyp wiedergegeben ist.

In Figur 1 ist mit 10 insgesamt eine Prothese bezeichnet, die in den Oberschenkel eines Patienten zur Bildung eines künstlichen Hüftgelenkes eingegliedert wird. Die Prothese 10 hat einen insgesamt mit 12 bezeichneten Prothesenkörper mit einer abgewinkelten Tragplatte 14. Deren Oberseite trägt einen nach medial gekippten Tragzapfen 16, auf welchem eine Gelenkkugel 18 angebracht ist.

Die distale Begrenzungsfläche der Tragplatte 14 sitzt auf einem insgesamt mit 20 bezeichneten Verankerungsabschnitt, der nach Art eines Käfigs aufgebaut ist. Dieser umfaßt im wesentlichen die Ecken einer sich nach distal verjüngenden und rechteckigen Querschnitt aufweisenden Pyramide bildende Hauptverankerungspfeiler 22, die durch Nebenverankerungspfeiler 24 verbunden sind, welche in der Praxis zickzackförmig zwischen benachbarten Hauptverankerungspfeilern 22 verlaufen können.

Die Hauptverankerungspfeiler 22 und die Nebenverankerungspfeiler 24 tragen jeweils axial beabstandete Verankerungsbunde 26, die in der Praxis 1 bis 2 mm über den Pfeilerkern überstehen. Die axiale Abmessung der Verankerungsbunde 26 kann etwa 1 bis 2 mm betragen, ihr axialer Abstand etwa 3 bis 6 mm.

Der Prothesenkörper ist ein Gußteil aus einer biokompatiblen Legierung oder ein Schmiedeteil, welches auf einer NC-Maschine spanend nachbearbeitet ist.

Bei der in den Figuren 3 und 4 gezeigten einzuzementierenden Prothese sind Prothesenteile, die in funktionsgleicher Form schon obenstehend unter Bezugnahme auf die Figuren 1 und 2 erläutert wurden, wieder mit analogen Bezugszeichen versehen, denen jedoch, soweit Unterschiede bestehen, zur Unterscheidung ein "*" angehängt ist. Diese Prothesenteile werden nachstehend nicht mehr im einzelnen beschrieben.

Bei dem Prothesenkörper nach den Figuren 3 und 4 ist der zwischen den Hauptverankerungspfeilern 22 liegende Raum ausgefüllt, derart, daß die Hauptverankerungspfeiler 22 nur noch als Wulste 22* auf einem glattflächigen Kern erscheinen. In Abwandlung kann man die Mantelflächen der ehemaligen Hauptverankerungspfeiler 22 auch durch zu den letzteren tangentiale seitliche Begrenzungsflächen verbinden, so daß die Hauptverankerungspfeiler 22 nur noch in Form von Abrundungen des im wesentlichen rechteckigen, sich zum distalen Ende des Verankerungsabschnittes 20* hin verjüngenden tranversalen Querschnittes des Verankerungsabschnittes 20* erscheinen.

Wie die Zeichnungen zeigen, ist die Achse des Tragzapfens 18 in der durch die Proximalrichtung und die Lateralrichtung aufgespannten Ebene (Zeichenebene von Figur 1) um einen Winkel a gegen die Achse des unteren Endabschnittes des Verankerungsabschnittes 20 bzw. des Schaftes 20* verkippt. Der Winkel a beträgt beim dargestellten Ausführungsbeispiel etwa 35°. Sie ist ferner in der durch die Proximalrichtung und die Dorsalrichtung vorgegebenen Ebene (Zeichenebene von Figur 2) gegen den unteren Endabschnitt von Verankerungsabschnitt 20 bzw. Schaft 20* um einen Winkel b verkippt. Dieser beträgt beim dargestellten Ausführungsbeispiel etwa 15 °.

Wie schon aus dem Vergleich der Figuren 1 bis 4 ersichtlich, haben die dort gezeigten Prothesentypen teilweise identische (oberer Prothesenteil), teilweise geometrisch sehr ähnliche (Verankerungsabschnitt) lichte Außenkontur.

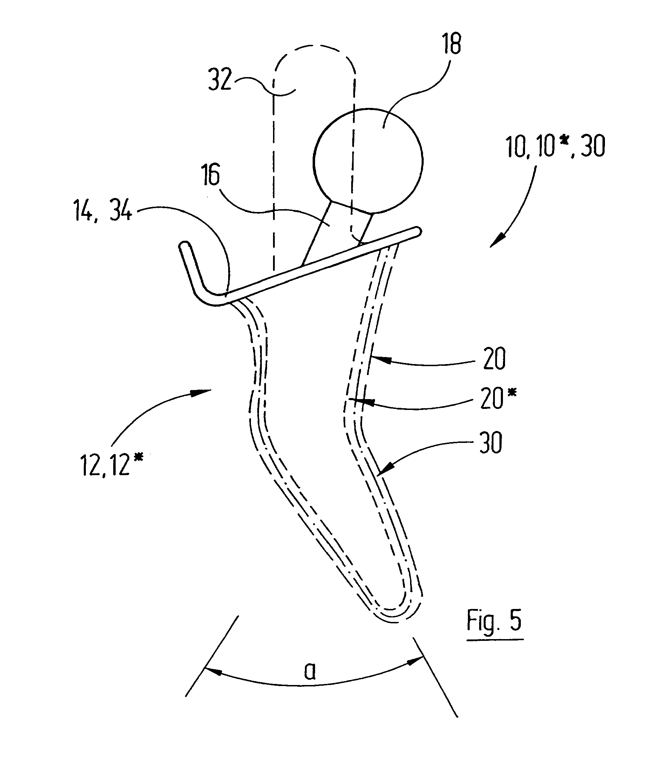

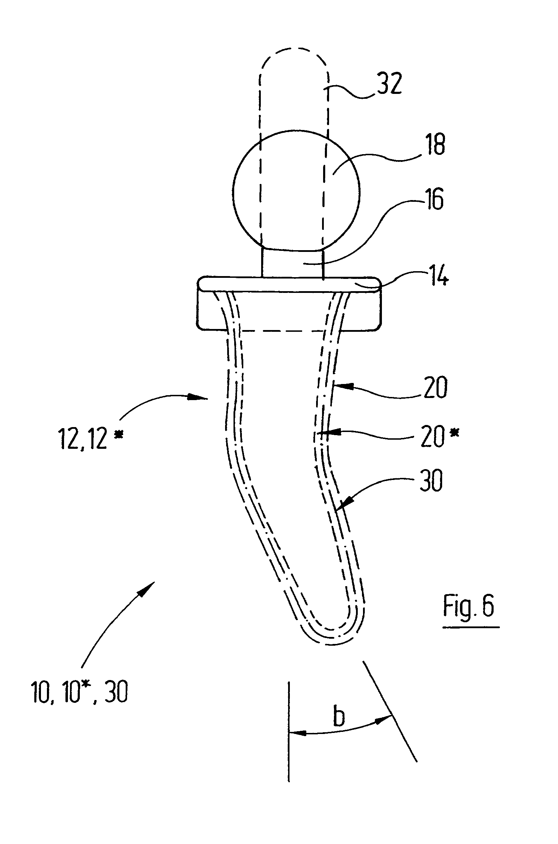

Wie in den Figuren 5 und 6 näher dargestellt, verlaufen die Außenkonturen der Verankerungsabschnitte 20, 20* von zementfrei zu im plantierenden Prothese 10 und zementierter Prothese 10* unter einem kleinen im wesentlichen konstanten Abstand d parallel zueinander. In der Praxis beträgt dieser Abstand etwa 1 bis 2 mm, vorzugsweise etwa 1,5 mm. Im übrigen haben die beiden Prothesentypen gleich Geometrie, das heißt, ihre Tragplatten 14, ihre Tragzapfen 16 und auch die von den letzteren getragenen Gelenkkugeln 18 sind gleich.

Die Implantierungsvorbereitungen für beide Prothesentypen sind identisch: Der Femur, in dessen oberes Ende die Prothese eingesetzt werden soll, wird so reseziert, daß der Trochander minor mit der verschlissenen Gelenkkugel vollständig entfernt wird, ein Teil des Trochander major dagegen stehenbleibt. Die Resektionslinie entspricht der Kontur der Tragplatte 14.

Aus dem so resezierten Knochenende wird die Spongiosa mit einem Löffel oder dergleichen ausgeräumt, daß sie grob der Geometrie eines Verankerungsabschnittes 20 entspricht, jedoch Untermaß aufweist.

Diese roh bearbeitete Kavität wird mit einem Endbearbeitungswerkzeug 30 spanend bearbeitet, dessen lichte Außenkontur in Figur 5 und 6 strichpunktiert angedeutet ist. Danach liegt die lichte Außenkontur des Endbearbeitungswerkzeuges beim betrachteten Ausführungsbeispiel durchweg etwa in der Mitte zwischen den beiden Verankerungsabschnitten 20 von zementfrei zu implantierender Prothese und einzuzementierender Prothese. Der Abstand zwischen der lichten Außenkontur des Endbearbeitungswerkzeuges 30 von der lichten Außenkontur der zementiertem Prothese entspricht allgemein der Dicke der später gewünschten Zementschicht (vorzugsweise etwa 1,5 mm), der Abstand der lichten Außenkontur des Endbearbeitungswerkzeuges 30 von der lichten Außenkontur der zementfrei zu implantierenden Prothese der Stärke des Preßsitzes des Verankerungsabschnittes 20 in der Corticalis des Femurs und beträgt vorzugsweise etwa 0,5 mm.

In Figur 5 und 6 ist bei 32 ein Griff des Endbearbeitungswerkzeuges 30 dargestellt, und bei 34 ist eine Formschlußplatte gezeigt, die zur Tragplatte 14 formgleich ist und dazu dient, das Endbearbeitungswerkzeug an der Resektionslinie des Femurs zu positionieren.

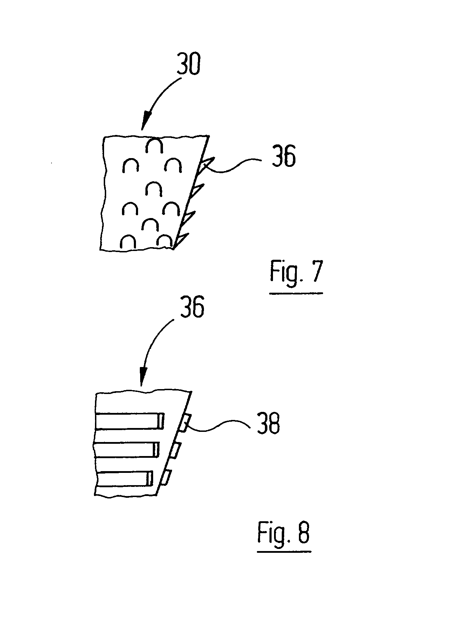

Das Endbearbeitungswerkzeug 30 kann gemäß Figur 7 aus einem Rohling hergestellt sein, dessen Außenkontur der lichten Außenkontur der zementierten Prothese entspricht und aus dem durch Hiebe einzelne Raspelzähne 36 nach außen ragend erzeugt wurden, deren Spitzen die lichte Außenkontur des Endbearbeitungswerkzeuges 30 vorgeben.

Gemäß Figur 7 kann das Endbearbeitungswerkzeug 30 auch aus einem hohlen Rohling hergestellt sein (der seinerseits z.B. durch Tiefziehen von Blech hergestellt ist), aus dem dann durch Erzeugen von Einschnitten und Biegen der den Einschnitten benachbarten Materialbereiche Hobelklingen 38 parallel herausgedrückt wurden, deren Schneiden die lichte Außenkontur des Endbearbeitungswerkzeuges 30 vorgeben.

## Patentansprüche

1. Satz aus Prothesen (10) unterschiedlicher Größe,
die jeweils aufweisen: eine Tragplatte (14), an welcher ein Gelenkelement (18) angebracht ist, und einen verankerungsabschnitt (20), der in eine Kavität eines Knochens (28) einführbar ist, wobei der Prothesensatz mindestens ein Paar von Prothesen (10, 10*) aufweist, von denen eine erste Prothese (10) einen zementfrei implantierbaren Verankerungsabschnitt (20) aufweist, und von denen eine zweite, mittels Knochenzement implantierbare Prothese (10*) einen als massiver Schaft ausgebildeten Verankerungsabschnitt (20*) aufweist, wobei die lichte Außenkontur des Verankerungsabschnitts (20) der ersten Prothese (10) unter im wesentlichen konstanten Abstand parallel verlaufend über der lichten Außenkontur des verankerungsabschnitts (20*) der zweiten Prothese (10*) liegt,
**dadurch gekennzeichnet, daß**
der Verankerungsabschnitt (20) der ersten Prothese (10) als Käfig ausgebildet ist,
die Geometrien der Verankerungsabschnitte (20, 20*) der Corticalis eines Oberschenkelknochens gut derart angepaßt sind, daß zwischen ihrer lichten Außenkontur und der Innenfläche der durch die Corticalis begrenzten Kavität ein im wesentlichen über die Kavität hinweg konstanter Abstand besteht und
die Verankerungsabschnitte (20, 20*) beider Prothesenteile (20, 20*) einen in den Trochanter major ragenden Abschnitt aufweisen.

2. Prothesensatz nach Anspruch 1, **dadurch gekennzeichnet,**
**daß** der Abstand zwischen den beiden lichten Außenkonturen der Verankerungsabschnitte (20, 20*) von erster Prothese (10) und zweiter Prothese (10*) 1 bis 3 mm, vorzugsweise etwa 1,5 bis 2 mm beträgt.

3. Prothesensatz nach Anspruch 1 oder 2, **dadurch gekennzeichnet, daß** der transversale Querschnitt der lichten Außenkonturen der Verankerungsabschnitte (20, 20*) im wesentlichen rechteckigen Querschnitt hat, wobei die Ecken des Rechteckes vorzugsweise abgerundet sind.

4. Prothesensatz nach einem der Ansprüche 1 bis 3,
**dadurch gekennzeichnet, daß** der Querschnitt der verankerungsabschnitte (20, 20*) zum distalen Ende der Verankerungsabschnitte hin abnimmt.

5. Prothesensatz nach einem der Ansprüche 1 bis 4,
**dadurch gekennzeichnet, daß** die Verankerungeabschnitte (20, 20*) jeweils mindestens einen um ihre Längsachse tordierten Unterabschnitt aufweisen.

6. Prothesensatz nach einem der Ansprüche 1 bis 5,
bei welchem die verankerungsabschnitte (20, 20*) jeweils eine Hauptfläche aufweisen, die im wesentlichen durch die Proximalrichtung und die Lateralrichtung aufgespannt ist, **dadurch gekennzeichnet, daß** die Verankerungsabschnitte (20, 20*) jeweils einen proximalen Unterabschnitt und einen distalen Unterabschnitt aufweisen, die senkrecht zur Hauptfläche gesehen gegeneinander verkippt sind.

7. Prothesensatz nach einem der Ansprüche 1 bis 6, **dadurch gekennzeichnet, daß** er ferner ein Werkzeug (30) zum spanenden Endbearbeiten der Innenfläche einer in einem Knochenende erzeugten Kavität umfaßt, in welche ein Verankerungsabschnitt einer Prothese eingeführt werden soll, wobei die lichte Außenkontur des Endbearbeitungswerkzeuges (30) unter im wesentlichen konstantem Abstand über der lichten Außenkontur der zweiten Prothese (10*) und unterhalb der lichten Außenkontur der ersten Prothese (10) liegt.

8. Prothesensatz nach Anspruch 7, **dadurch gekennzeichnet, daß** die lichte Außenkontur des Endbearbeitungswerkzeugs (30) in der Mitte zwischen den lichten Außenkonturen von erster Prothese (10) und zweiter Prothese (10*) liegt.

9. Prothesensatz nach Anspruch 7 oder 8, **dadurch gekennzeichnet, daß** das Endbearbeitungswerkzeug (30) als Raspel ausgebildet ist, die durch Einbringen von Hieben in einen Werkzeugrohling hergestellt wurde, wobei letzterer eine der lichten Außenkontur des Verankerungsabschnittes (20*) einer zweiten Prothese (10*) entsprechende Außenfläche aufweist.

10. Prothesensatz nach Anspruch 7 oder 8, **dadurch gekennzeichnet, daß** das Endbearbeitungswerkzeug (30) hohl ist und durch Ausstanzen und Ausbiegen von Klingenabschnitten (38) aus einem hohlen Rohling hergestellt wurde, dessen Außenkontur der eines Verankerungsabschnittes (20*) einer zweiten Prothese (10*) entspricht.

## Claims

1. A set of prostheses (10) of different sizes, which
each comprise: a support plate (14), to which a condyle joint element (18) is fitted, and an anchoring section (20), which can be introduced into a cavity of a bone (28), wherein the set comprises at least one pair of prostheses (10, 10*), of which a first prosthesis (10) comprises an anchoring section (20) which is to be implanted in a cement-free manner, and of which a second prosthesis (10*) comprises an anchoring section (20*) constructed as a solid shaft, the clear external contour of the first prosthesis (10) lying at a substantially constant distance beyond and parallel to the clear external contour of the second prosthesis (10*), **characterized in that** the anchoring section (20) of the first prosthesis (10) is formed as a cage, and **in that** the geometries of the anchoring sections (20, 20*) are conformed to the corticalis of a femur that well that between the clear external contour of the prostheses and the interior surface of the cavity defined by the corticalis there is an essentially constant distance throughout the cavity and **in that** the anchoring sections (20, 20*) of both prostheses (10, 10*) have portions projecting into the trochanter major.

2. A prosthesis set as claimed in claim 1, **characterized in that** the distance between the two clear external contours of the first prosthesis (10) and second prosthesis (10*) measures 1 to 3 mm, preferably approximately 1.5 to 2 mm.

3. A prosthesis set as claimed in claim 1 or 2, **characterized in that** the transverse cross section of the clear external contour of the anchoring sections (20, 20*) has a substantially rectangular cross section, the corners of the rectangle preferably being rounded.

4. A prosthesis set as claimed in one of claims 1 to 3, characterrized in that the cross section of the anchoring sections (20, 20*) decreases towards the distal end of the anchoring sections.

5. A prosthesis set as claimed in one of claims 1 to 4, wherein **characterized in that** the anchoring sections (20, 20*) each comprise at least one lower section twisted about its longitudinal axis.

6. A prosthesis set as claimed in one of claims 1 to 5, in which the anchoring sections (20, 20*) each comprise a main surface area, which is essentially prescribed by the proximal direction and the distal direction, **characterized in that** the anchoring sections (20, 20*) each comprise a proximal lower section and a distal lower section, shich in turn comprise lower main surface areas, and the lower main surface areas of the lower sections of the anchoring sections are each tilted relative to one another as seen in a direction being perperdicular to the main surface.

7. A prosthesis set as claimed in one of claims 1 to 6, **characterized in that** it comprises a tool (30) for chip forming finishing the internal surfaces of a cavity produced in a bone end, into which an anchoring section of a prosthesis is to be introduced, **characterized in that** the clear external contour of the finishing tool (30) lies at a substantially constant distance beyond the clear external contour of the second prosthesis (10*) and short of the clear external contour of the first prosthesis (10).

8. A prosthesis set as claimed in claim 7, **characterized in that** its clear external contour lies in the centre between the clear external contours of the first prosthesis (10) and second prosthesis (10*).

9. A prosthesis set as claimed in claim 7 or 8, **characterized in that** the finishing tool (30) is constructed as a rasp, which has been manufactured by making cuts in a tool blank, the latter comprising an external surface corresponding to the clear external contour of the anchoring section (20*) of a second prosthesis (10*).

10. A prosthesis set as claimed in claim 7 or 8, **characterized in that** the finishing tool (30) is hollow and has been manufactured by punching and bending blade sections (38) from a hollow blank, whose external contour corresponds to that of an anchoring section (20*) of a second prosthesis (10*).

## Revendications

1. Jeu de prothèses (10) de taille différente et comprenant chacune: une plaque de support (14), à laquelle est fixé un élément d'articulation (18), et une section d'ancrage (20) pouvant être introduite dans une cavité d'os (28), le jeu de prothèses comprenant du moins une paire de prothèses (10, 10*) dont une première prothèse (10) comprend une section d'ancrage pouvant être implantée sans ciment et dont une deuxième prothèse (10*) pouvant être implantée faisant recours à un ciment à os comprend une section d'ancrage (20*) formé comme tige massive, le contour circonscrit extérieur de la section d'ancrage (20) de la première prothèse (10) s'étendant à distance essentiellement constante de manière parallèle devant le contour circonscrit extérieur de la section d'ancrage (20*) de la deuxième prothèse (10*), charactérisée en ce que la section d'ancrage (20) de la première prothèse (10) est formée comme cage, que les géométries des sections d'ancrage (20, 20*) sont adaptée à la corticalis d'un femur tellement bien qu'il y a entre le contour circonscrit extérieur de la prothèse et la surface interne de la cavité d'élimité par la corticalis une distance essentiellement constante sur toute la cavité et que les sections d'ancrage (20, 20*) des deux prothèses (10, 10*) comprennent une section projetant dans le trochanter major.

2. Jeu de prothèses selon la revendication 1, charactérisé en ce que la distance entre les deux contours circonscrits extérieurs des sections d'ancrage (20, 20') de la première prothèse (10*) et de la deuxième prothèse (20*) est de 1 à 3 mm, de préférence de 1,5 à 2 mm.

3. Jeu de prothèses selon la revendication 1 ou 2, charactérisé en ce que la section transversale du contour extérieur des sections d'ancrage (20, 20*) est essentiellement de section rectangulaire, les coins du rectangle étant arrondis, de préférence.

4. Jeu de prothèses selon une des revendications 1 à 3,
charactérisé en ce que la section des sections d'ancrage (20, 20*) diminue vers l'extrémité distale des sections d'ancrage.

5. Jeu de prothèses selon une des revendications 1 à 4,
charactérisé en ce que les sections d'ancrage (20, 20*) chacune comprennent du moins une sous-section étant tordue par rapport à leur axe longitudinale.

6. Jeu de prothèses selon une des revendiactions 1 à 5,
dans laquelle les sections d'ancrage (20, 20') chacune comprennent une face principale essentiellement définie par le directions proximales et latérales, charactérisée en ce que les sections d'ancrage (20, 20') chacune comprennent une sous-section proximale et une sous-section distale étant mutuellement inclinées vu dans une direction perpendiculaire à la face principale.

7. Jeu de prothèses selon une des revendications 1 à 6, charactérisé en ce qu'il comprend également un outil (30) de finition travaillant en enlèvement de copaux de la surface interne d'une cavité produite dans une extrémité d'os qui doit recevoir la section d'ancrage d'une prothèse, le contour extérieur de l'outil (30) de finition s'étendant à distance essentiellement constante à l'extérieur du contour circonscrit extérieur de la deuxième prothèse (10*) et au-dessous du contour circonscrit extérieur de la première prothèse (10).

8. Jeu de prothèses selon la revendication 7, chactérisé en ce que le contour circonscrit extérieur de l'outil de finition (30) se trouve au milieu entre les contours circonscrits extérieurs de la première prothèse (10) et de la deuxième prothèse (10*).

9. Je de prothèse selon la revendication 7 ou 8, charactérisé en ce que l'outil de finition (30) est formé comme râpe produite par formation des tailles dans une pièce brute, la dernière ayant une surface extérieure correspondant au contour circonscrit extérieur de la section d'ancrage de la deuxième prothèse (10*).

10. Jeu de prothèses selon la revendication 7 ou 8, charactérisé en ce que l'outil de finition (30) est creux et a été produit par stampage et pliage des sections de couteaux (38) d'une pièce brute creuse, le contour extérieur de la dernière correspondant au contour circonscrit extérieur d'une section d'ancrage (20*) d'une seconde prothèse (10*).
